# EUROPEAN PATENT APPLICATION

(11) **EP 2 687 845 A1**
(43) Date of publication of application: **22.01.2014**
(21) Application number: 12177333.7
(22) Date of filing: 20.07.2012
(51) Int. Cl.: G01N 33/50, G01N 33/68

(54) **Fucose as a biomarker for gut immunity**

(71) Applicant: Nestec S.A., 1800 Vevey (CH)
(72) Inventor: Genick, Ulrich Karl, 2520 La Neuveville (CH); Ledda, Mirko Aurelio, 1202 Geneva (CH); Montoliu Roura, Ivan, 1000 Lausanne 26 (CH); Le Coutre, Johannes, 1009 Pully (CH); Rezzi, Serge André Dominique, 1623 Semsales (CH); Collino, Sebastiano, 1003 Lausanne (CH); Martin, François-Pierre, 1305 Penthalaz (CH); Da Silva, Laeticia, 1432 Belmont-sur-Yverdon (CH)
(74) Representative: Kamibayashi, Lynne

(57) **Abstract**

The present invention generally relates to the field of biomarkers. For example, the present invention relates to biomarkers that can be used to assess the likelihood of having the FUT2 secretor genotype in a subject. The invention also relates to biomarkers that can be used to assess gut health. Fucose was identified as biomarker that can be used for these purposes.

## Description

The present invention generally relates to the field of biomarkers. For example, the present invention relates to biomarkers that can be used to assess the likelihood of having the FUT2 secretor genotype in a subject. The invention also relates to biomarkers that can be used to assess gut health. Fucose was identified as biomarker that can be used for these purposes.

Digestive health and comfort are intimately linked to the proper balance of microbiotic species that colonize the digestive tract [ Round JL, Mazmanian SK (2009) Nat Rev Immunol 9: 313-323]. A disturbance of this balance has been linked to a number of conditions that range from discomfort to debilitating disease (e.g. Crohn's disease). Gut-microbial composition in turn depends on multiple factors including diet and lifestyle, but genetic predisposition is increasingly recognized as a critically important factor. Among the genetic factors the FUT2 gene plays a central role [ McGovern DP, et al., (2010) Hum Mol Genet 19: 3468-3476].

The FUT2 gene encodes a fucosyl transferase enzyme that catalyzes the attachment of a fucosyl moiety to the growing H Type-1 antigen, which is a key step in the assembly and subsequent secretion of ABO-antigens into mucosal layers [ Lindesmith L, et al. (2003) Nat Med 9: 548-553]. The FUT2 gene exists in two basic forms - an active one (the so-called "secretor" form) and an inactive one (the so-called "non-secretor" form). Individuals who carry at least one copy of the secretor form secrete the ABO-antigens and display them on mucosal surfaces, including the mucosal lining of the gut. In individuals who carry only non-secretor versions of the FUT2 gene the production and secretion of the ABO-antigens is blocked and no ABO-antigens are found in the mucosal layers. The main molecular determinant for secretor phenotype was traced to a C/T single nucleotide polymorphism (rs601338) in the protein-coding region of the FUT2 gene. The C-to-T change converts the codon for amino acid 143 from a tryptophan to a stop codon, which results in a non-functional protein for the T-variant [ Lindesmith L, et al. (2003) Nat Med 9: 548-553]. And, as a result, individuals who carry two copies of the non-secretor variant of the FUT2 gene don't produce the functional fucosyl-transferase and do not present ABO-antigens on mucosal surfaces.

The effects of the FUT2 polymorphisms on gastro-intestinal health are thought to occur because the oligo-saccharide structures of the ABO-antigen serve as attachment points for a number of microorganisms. On the one hand, the presence of the ABO-antigen appears to favor colonization of the human digestive tract by beneficial bacteria [ McGovern DP, et al., (2010) Hum Mol Genet 19: 3468-3476, Rausch P, et al., (2011) Proc Natl Acad Sci U S A 108: 19030-19035] and, as a consequence, reduces the risk of excessive inflammatory responses. In this context the secretor form of the FUT2 gene has a protective function and individuals who carry at least one copy of the secretor version of FUT2 are less likely to develop problems involving inflammatory responses of the gut. On the other hand, the same ABO-antigens also serve as attachment points for pathogenic viruses including the Norwalk virus [ Lindesmith L, et al. (2003) Nat Med 9: 548-553]. In susceptible individuals this virus causes severe gastro intestinal distress with the potential for severe health consequence in elderly and immune compromised individuals. In this context the non-secretor form of the FUT2 gene is beneficial. Individuals who carry two copies of the non-secretor form of FUT2 do not provide attachment points for the virus and are therefore virtually immune to Norwalk virus infections.

In either context knowing if an individual has a secretor or a non-secretor FUT2 genotype can provide important information in the diagnosis of gastrointestinal problems but more importantly it enables a proactive management of potential risks through diet or lifestyle adjustments.

Currently FUT2 secretor status is being determined via genetic testing. Despite advances in genotyping technology, genetic testing still requires specialized laboratory equipment and a substantial amount of time. As a result, genetic testing is generally not possible in local diagnostic labs and requires mailing of samples to a central laboratory. This procedure generates a substantial time delay between the time of sample collection and the time where results are available. More importantly genetic testing provides a high emotional hurdle for many individuals. As a result individuals may avoid determination of their FUT2 secretor status even though they could benefit from this information.

Consequently, it was the objective of the present invention to improve the state of the art and in particular to provide a simple, rapid and low-cost method to assess the likelihood of a person's secretor or non-secretor status for FUT2. Ideally this method should be non-invasive and/or can be self-administered.

The present inventors have addressed this need and were surprised to be able to achieve this objective by the subject matter of the independent claims. The dependant claims further develop the idea of the present invention.

The inventors have worked on a method to determine a person's genetic predisposition for gastro-intestinal problems and were surprised to find that the total fucose level, e.g., in urine, is strongly linked to FUT2 secretor status. This link between FUT2 secretor status and fucose levels in urine was not expected *a priori.* Previous to their observations FUT2 was thought to be involved in the secretion of the fucose-containing H antigen into mucosa but not implicated in the urine metabolism of fucose.

The present invention allows it now, for example, to determine the likelihood of having the FUT2 secretor genotype in a subject by measuring the concentration of the biomarker Fucose in that person's urine. This invention avoids the need for gene-based testing and provides a fast and simple route for assessing FUT2 secretor status via rapid, low-cost and/or self-administered tests for Fut2 secretor status.

In particular, the present invention provides a non-invasive (urine-based) technique to determine the likelihood that a person carries a variant of the FUT-2 gene that increases his/her susceptibility to specific gastro intestinal health risks.

The inventors have used an untargeted genome-wide association study of the human urine metabolome, and have identified a biomarker that is very strongly correlated with the FUT2 secretor genotype.

This biomarker is fucose. The FUT2 secretor genotype has, in turn, shown a strong association with the composition of the human gut microbiota (Wacklin P, et al. (2011) PLoS ONE 6(5), e20113) and, e.g., with incidence for type 1 diabetes (Yang et a. (2011) DIABETES, VOL. 60, 2685).

Due to the strength of the association between the new biomarker fucose and the FUT2 secretor genotype on the one hand and the FUT2 secretor genotype and the gut microbiota on the other hand, the inventors propose to use fucose as biomarker, e.g. in urine, for example as an indication of the gut-microbial composition.

Hence, using fucose as a biomarker, e.g., in urine provides a simple and cheap test for assessing the gut microbial state of subjects to be tested.

Consequently, the present invention relates in part to a biomarker for gut health, wherein the biomarker is fucose.

The present invention also comprises the use of fucose as a biomarker for gut health.

This diagnostic method is practiced outside of the human or animal body.

The present invention also comprises fucose for use in a diagnostic method for determining the likelihood of having the FUT2 secretor genotype and/or the risk of developing disorders associated therewith.

Irrespective of the chosen body fluid, the subject matter of the present invention has the advantage that obtaining such body fluids from a subject is a well established procedure.

The actual diagnosis is then carried out in a body fluid sample outside the body.

Typically, the biomarker detection and/or quantification step is carried out in a body fluid sample that was previously obtained from the subject to be tested.

The fucose may be bound fucose. Fucose is often present as bound fucose, since fucose frequently found in nature covalently attached to glycoproteins at the cell surface. Consequently, "Bound fucose" is fucose linked to protein or sugar residues. This link may be covalent.

The fucose may also be free fucose.

The subject matter of the present invention will work irrespective of whether bound fucose or free fucose is assessed.

For example, also total fucose concentration can be measured as the sum of free and bound fucose concentration.

The fucose may be L-fucose. L-Fucose (also named Isodulcit) is the fucose enantiomer that is widely occurring in nature.

The detection and/or quantification of fucose as biomarker may be carried out in any body fluid. For the subject matter of the present invention, the body fluid may be blood, blood plasma, blood serum or urine, for example.

Urine has the advantage that the body fluid sample can be obtained non-invasively. Hence, the biomarker may be to be detected in urine.

The present invention extends to a method for determining the likelihood of having the FUT2 secretor genotype in a subject, comprising determining the level of fucose in a body fluid sample previously obtained from the subject to be tested, and comparing the subject's fucose level to a predetermined reference value. The predetermined reference value may be based on an average body fluid fucose level in a control population. A lower body fluid fucose level in the sample compared to the predetermined reference value indicates an increased likelihood for a non-secretor FUT2 genotype and an increased body fluid fucose level compared to the predetermined reference value indicates an increased likelihood for a secretor FUT2 genotype.

The level of fucose in the sample can be detected and quantified by any means known in the art. For example, mass spectroscopy, e.g, UPLC-ESI-MS/MS, or NMR spectroscopy, e.g. ¹H-NMR spectroscopy, may be used. Other methods, such as other spectroscopic methods, chromatographic methods, labeling techniques, antibody based methods such as ELISA, or quantitative chemical methods may be used as well.

Ideally, the fucose level in the sample and the reference value are determined by the same method.

It is further preferred if the fucose levels in the sample and the reference value are determined in the same body fluid.

This body fluid may be urine, for example.

The predetermined reference value may be based on an average fucose level in the tested body fluid in a control population. The control population can be a group of at least 3, preferably at least 10, more preferred at least 50 people with a similar age and/or average health status.

An advantage of the present invention is that the genetic background other than FUT2 genotype seems to not have a significant effect in the described association study. Similar gender seems to have no significant effect. This allows applying the predetermined reference values to a large number of people.

The control population can also be the same person, so that the predetermined reference value is obtained previously from the same subject. This will allow a direct comparison of the effect of a present lifestyle to a previous lifestyle on visceral adiposity, for example, and improvements can be directly assessed.

Optionally, the obtained fucose level can be corrected for one or more potential covariates such as age, gender, BMI, urine dilution, or alcohol consumption, for example. Such corrections will further increase the predictive power of the method proposed. Skilled artesians will be able to apply appropriate corrections.

The predetermined reference value may be obtained from a control population with a non-secretor FUT2 genotype. In this case, a higher fucose level in the sample compared to the predetermined reference value indicates a secretor FUT2 genotype while an equal or lower fucose level indicates a non-secretor FUT2 genotype.

Alternatively, the predetermined reference value may be obtained from a control population with a secretor FUT2 genotype. In this case, a lower fucose level in the sample compared to the predetermined reference value indicates a non-secretor FUT2 genotype and an equal or higher fucose level indicates a secretor FUT2 genotype.

Using directly a secretor genotype or a non-secretor genotype as predetermined reference values has the advantage that the fucose concentration in the body fluid of subjects with the opposite genoptype will differ more significantly from the reference value compared to a reference value obtained from a mixture of all FUT2 genotypes. This will increase the predictive power of the diagnosis method of the present invention.

The reference value for fucose is preferably measured using the same units used to characterize the level of fucose obtained from the test subject. Thus, if the level of fucose is an absolute value such as the units of fucose in µmol/l (µM) the reference value is also based upon the units of fucose in µmol/l (µM) in individuals in the general population or a selected control population of subjects.

Moreover, the reference value can be a single cut-off value, such as a median or mean. Reference values of fucose in obtained body fluid samples, such as mean levels, median levels, or "cut-off" levels, may be established by assaying a large sample of individuals in the general population or the selected population and using a statistical model such as the predictive value method for selecting a positivity criterion or receiver operator characteristic curve that defines optimum specificity (highest true negative rate) and sensitivity (highest true positive rate) as described in Knapp, R. G., and Miller, M. C. (1992). Clinical Epidemiology and Biostatistics. William and Wilkins, Harual Publishing Co. Malvern, Pa., which is incorporated herein by reference.

Skilled artesians will know how to assign correct reference values as they will vary with gender, race, genetic heritage, health status, urine dilution, or age, for example.

The FUT2 secretor genotype has recently attracted significant attention in science. For example, in Nature Reviews Gastroenterology & Hepatology 9, 2 (2012), Franks describes that the FUT2 (secretor) genotype is thought to have a general role in maintaining host-microbial homeostasis, as well as being associated with susceptibility to a variety of individual pathogens. In addition, the loss-of-function mutation W143X (G428A) is associated with increased susceptibility to Crohn's disease.

The subject matter of the present invention allows it for example to detect the FUT2 secretor genotype in a simple way by using the fucose concentration in a body liquid as a biomarker.

A non-secretor FUT2 genotype was found to correspond to an increased risk for an impaired gut health, for example to an increased susceptibility to inflammatory bowel disease, Crohn's disease or other chronic intestinal inflammatory processes.

Such other chronic intestinal inflammatory processes may be for example the selected from the group consisting of Inflammatory bowel disease**, gastritis, colitis, ascites or irritable colon,** or combinations thereof.

A non-secretor FUT2 genotype was also found to correspond to an increased susceptibility to Type 1 Diabetes.

Further, it was found that a non-secretor FUT2 genotype corresponds to an altered gut functional ecology and/or an increased risk towards gut dysbiosis.

An altered gut functional ecology comprises for example reduced bifidobacterial diversity, reduced bifidobacterial richness, and/or reduced bifidobacterial abundance.

A non-secretor FUT2 genotype was also found to correspond to a decreased risk for gastrointestinal virus infection. Thorven et al. report in the JOURNAL OF VIROLOGY, 2005, p. 15351-15355 that non-secretor FUT2 genotype provides resistance to symptomatic norovirus infections. Hence a non-secretor FUT2 genotype corresponds to a decreased risk for norovirus infection.

The subject matter of the present invention may also be used to identify subjects at risk of metabolic deregulations. Subjects with a non-secretor FUT2 genotype are more likely to be at risk of metabolic deregulations. Such metabolic deregulations may be for example **Type 1 diabetes.**

The subject matter of the present invention may further be used to stratify subjects according to their bifidobacterial population in the gut. Subjects with a non-secretor FUT2 genotype are more likely to have a less rich bifidobacterial culture in their intestinal tract than subjects with a secretor FUT2 genotype. Hence, it may be advisable for subjects with a non-secretor FUT2 genotype to take care that sufficient Bifidobacteria are consumed with their nutritional regimen. Hence, the present invention also relates to a method to stratify subjects according to their bifidobacterial population in the gut, comprising determining the level of fucose in a body fluid sample previously obtained from the subject to be tested, and comparing the subject's fucose level to a predetermined reference value, wherein the predetermined reference value is based on an average body fluid fucose level in a control population, and wherein an equal or higher body fluid fucose level in the sample compared to the predetermined reference value indicates a rich bifidobacterial population in the gut, while a lower body fluid fucose level in the sample compared to the predetermined reference value indicates an impaired bifidobacterial population in the gut.

Without wishing to be bound by theory, the inventors presently assume that the observed increased fucose levels in body fluids for subjects with a secretor FUT2 genotype might be at least partially caused by the richer bifidobacterial flora in the intestinal tract of these subjects. Changes in the gut microbiome will have an effect on the fucose levels in body fluids, and an improved gut microbiome will be reflected by increased fucose levels in body fluids. Hence, the subject matter of the present invention can be used to test the effectiveness of a diet, a nutritional product, a medicament or a new nutritional regimen in improving the gut flora.

The subject matter of the present invention has the advantage that it allows monitoring the effect of lifestyle changes on gut health, and altered gut functional ecology and/or on risks for associated disorders.

The change in lifestyle may be any change, such as a new job, a different stress level, a new relationship, increases or decreases in physical activity, and/or a change in overall wellbeing.

For example, the change in lifestyle may be a change in the diet.

The change in diet may be an increase or decrease in carbohydrate, lipid and/or protein content. It may be the switch to a different regional diet, such as the Mediterranean diet, for example. It may also be a change in total caloric intake.

As such the method of the present invention may be used to test the effectiveness of a new nutritional regimen, of nutritional products and/or of medicaments.

Nutritional products may be for example products that claim to have an effect on gut health, and altered gut functional ecology and/or on risks for associated disorders.

Typically, nutritional products may be food products, drinks, pet food products, food supplements, nutraceuticals, food additives or nutritional formulas.

For example, the change in the diet may be the use of at least one nutritional product that was previously not consumed or consumed in different amounts.

As such, the method of the present invention may be used to test the effectiveness of a new nutritional regimen and/or a nutritional product.

Consequently, the present invention also relates to a method to test the effectiveness of medical or nutritional products in improving the gut microbiome, in particular in improving the bifidobacterial gut population in a subject, comprising determining the level of fucose in a body fluid sample previously obtained from the subject to be tested, and comparing the subject's fucose level to a predetermined reference value, wherein the predetermined reference value is based on an average body fluid fucose level in a control population, and wherein a higher body fluid fucose level in the sample compared to the predetermined reference value indicates an improvement in the gut microbiome, in particular in the bifidobacterial gut population.

To increase accuracy of the measurements for the above applications where relative improvements are measured, it may be preferred to base the predetermined reference value on body fluid fucose levels obtained from the subject before the administration of the diet, a nutritional product, a medicament or a new nutritional regimen has started.

The subject matter of the present inventions may be applied to all subjects in need thereof, for example humans or animals. Typical animals may be mammals, for example companion animals such as cats or dogs. The subjects may be infants, children, adolescents, adults or elderly subjects, for example.

Those skilled in the art will understand that they can freely combine all features of the present invention described herein, without departing from the scope of the invention as disclosed. In particular, features described for the methods of the present invention may be applied to other methods and to the use of the present invention and vice versa.

Further advantages and features of the present invention are apparent from the following Examples and Figures.
Figure 1 shows a Manhattan plot resulting from a GWAS on the relative strength of the normalized urine ¹H-NMR signal at 1.256 ppm chemical shift, which has been assigned to L-fucose. The plot shows a single highly-significant association to the FUT2 locus on chromosome 19.
Figure 2 shows a QQ-plot derived from the Manhattan plot shown in figure 1. The plot underlines the strength of the observed association and confirms that no undue overall inflation of p-values was observed.
Figure 3 shows a Regression plot between genotype at SNP rs601338 and the normalized NMR signal at 1.256 ppm chemical shift. The figure shows that the genotypes indicative of secretor status (T/C and C/C) are associated with an increased NMR signal. Here a higher NMR signal corresponds to a higher level of L-Fucose in urine.
Figure 4 shows the p-value spectrum for rs601338 (see main text for further information). This p-value spectrum prominently features key spectral lines observed for reference samples of L-Fucose measured in the urine matrix.

### Examples

### Subject panel

The subject panel consisted of 600 healthy individuals aged 18-45 recruited from the general population of São Paulo Brazil. Subjects were selected to include equal numbers of males and females and to capture the pronounced ethnic mixture of African, European, Middle-Eastern and Asian ancestries that characterizes the São Paulo population. All procedures were approved by the Institutional Review Board of the Sirio Libanês Hospital, where tests were administered, and by the National Committee of Research Ethics at the Brazilian Ministry of Health (HSL 2007/25 Process no. 25000.114841/2007-17).

### Sample collection

Each subject gave three urine samples with 3-5 days between each sample donation. Urine samples were collected from the subjects in the morning at fasting state (before breakfast). Subjects were instructed to collect samples mid-stream. Sodium azide (3mM) was added as antimicrobial agent in the collected urine. Urine samples were agitated and aliquoted in 1mL cryo-resistant Eppendorf tubes and then stored at -80 deg. C.

Subjects also gave blood samples that were used to extract DNA for genotyping purposes.

### Genotyping

Genotyping was outsourced to Expression Analysis Inc. (Durham, NC, USA). Briefly, genomic DNA was extracted from whole blood and genotyping was performed on the Illumina Human Omni-Quad1 platform following standard protocols. Genotype calling was performed with Beadstudio software (Illumina). Calls with a genotyping score below 0.2 were excluded from further analysis. Single nucleotide polymorphisms (SNPs) with a call rate below 90% and individuals with a call rate below 95% were also excluded.

Genotyping data was of high quality with an average call rate of 99.8 % for all SNPs. 99.4% of SNPs had a call rate of greater than the cutoff value (95%) set for the rejection of individual SNPs. The average Q-score for all SNPs was 0.71 and for 99.6 % of called SNPs the Q-score passed the cutoff (0.2) for inclusion.

### Urine Sample Preparation and ¹H NMR Spectroscopic Analysis

Urine samples (200 µL) were adjusted to pH 6.8 using 400 µL of a deuterated phosphate buffer solution (KH₂PO₄, 0.2 M final concentration) containing 1 mM of sodium 3-(trimethylsilyl)-[2, 2, 3, 3-2H₄] -1-propionate (TSP) into 5 mm NMR tubes. A Bruker Avance II 600 MHz spectrometer equipped with a 5 mm inverse probe at 300 K (Bruker Biospin, Rheinstetten, Germany) was used for data collection. Urine ¹H NMR spectra were acquired using a standard ¹H detection pulse sequence with water suppression, using a relaxation delay of 2.5 s and a mixing time of 100 ms, as previously reported[ Rezzi S, et al., (2007) Journal of Proteome Research 6: 4469-4477]. For each urine sample, 128 Free Induction Decays (FIDs) were collected into 64 K data points using a spectral width of 12019.2 Hz and an acquisition time of 2.7 s. The free induction decays were multiplied by an exponential weighting function corresponding to a line broadening of 0.3 Hz before Fourier transformation. The acquired NMR spectra were manually corrected for phase and baseline distortions, and referenced to the chemical shift of TSP at δ 0.0 using the TOPSPIN (version 2.1, Bruker Biospin, Rheinstetten, Germany) software package.

The NMR spectra were converted into 12 K data points over the range of δ 0.4-10.0 and imported to MATLAB environment (The MathWorks Inc., Natick, MA, USA) excluding the water residue signal in between δ 4.7000-4.9992. NMR spectra were also normalized to the sum of all intensities within the specified range. Prior to data analysis, binning in segments of 0.0032 ppm was applied to correct for peak misalignment. This procedure consisted in the substitution of the intensity values of each segment in each spectrum by the integral of the intensity over that spectral range so that the NMR spectrum of each sample is represented by 2400 spectral bins.

Metabolite identification was achieved using literature data[ Nicholson (1995) Analytical Chemistry 67: 793-811], and confirmed by 2D ¹H NMR spectroscopy experiments performed on selected samples.

### Genome wide association study (GWAS)

The binned NMR spectra were prepared for genotype-metabotype association analysis by log-averaging the intensity values for each of the 2400 spectral bins across the three samples collected per subject. The raw intensity values were exponentially distributed and were therefore log-transformed prior to averaging. The NMR spectra had been collected in two separate batches of 300 subjects each. To minimize batch effects the NMR intensity values were normalized within their respective batches and the z-prime scores were merged and used as the input phenotype for GWAS analysis.

Genotype-metabotype analysis was carried out as 2400 parallel GWAS studies using the subjects' z-prime values for a given spectral bin as the input phenotype. Multiple linear regression was used to identify and correct significant covariates (age, BMI, gender and the 10 first principal components of a genetic ancestry PCA analysis) for each of the 2400 input phenotype (i.e. each spectral bin) separately.

The individual GWASs were performed using a linear allele dosage model implemented as in-house code in the MATLAB environment (The MathWorks Inc., Natick, MA, USA) followed by genomic control.

Associations were considered to be statistically significant if they achieved a p-value below 1.3 x 10⁻¹¹, which corresponds to the standard genome-wide-significance criterion (p-value < 10^{-7.5}) after Bonferroni correction for the number (2400) of parallel GWASs.

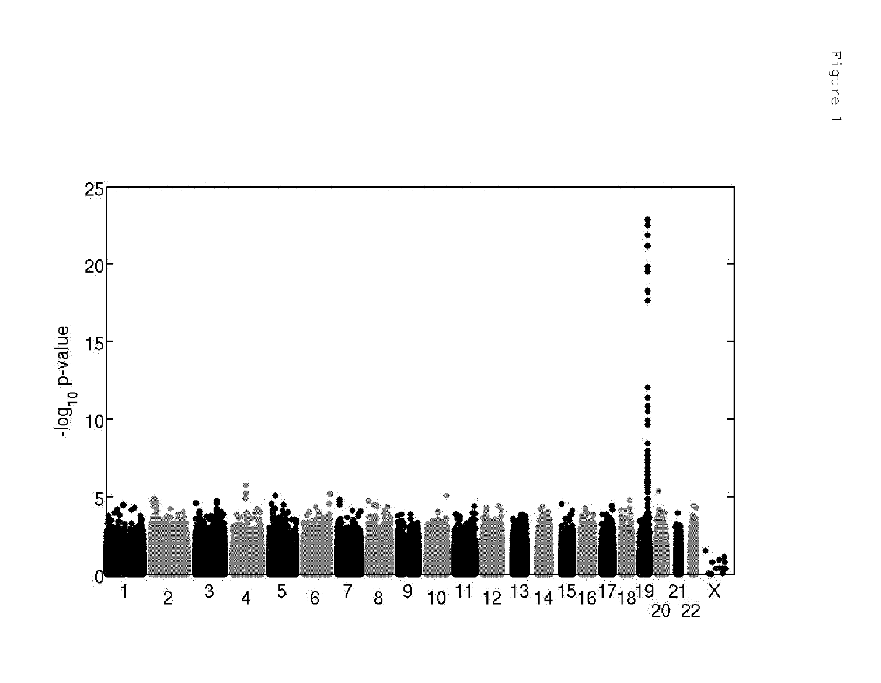

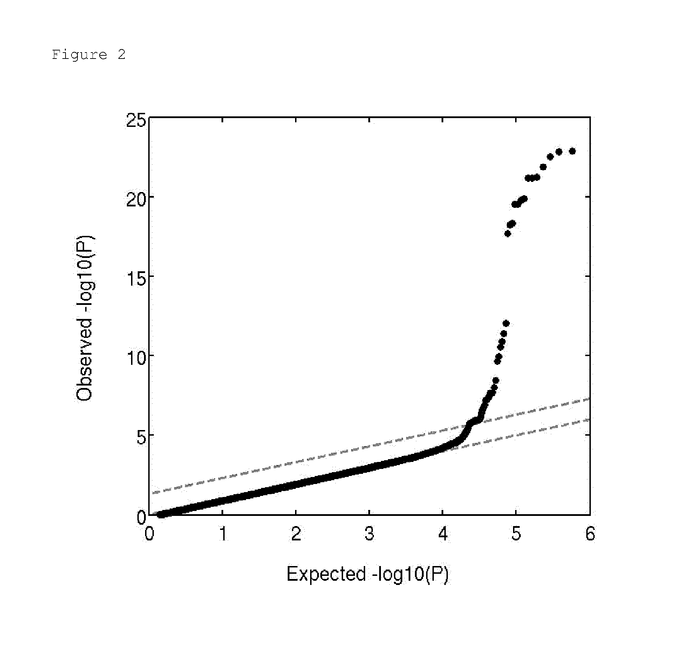

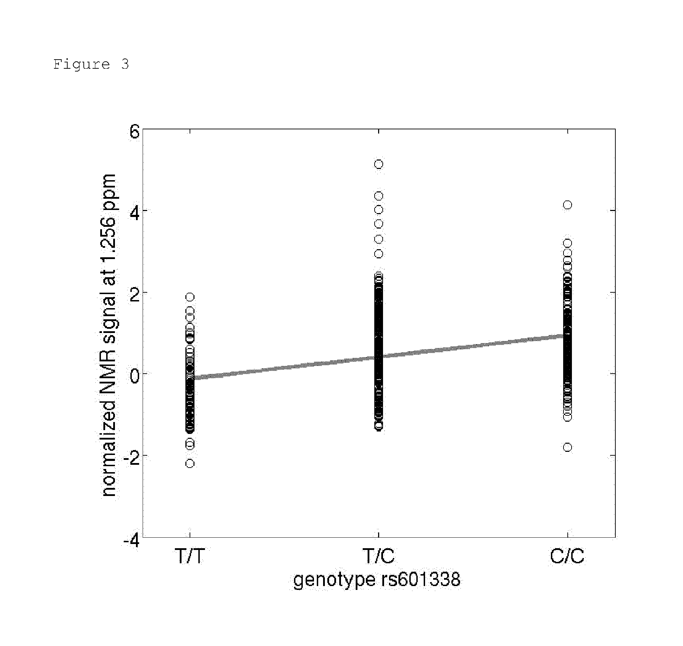

Figure 1 shows the Manhattan plot resulting from the GWAS conducted with the NMR signal at a chemical shift of 1.256 ppm. The strong association signal (p-value < 10⁻²²) on chromosome 19 falls squarely on the FUT2 locus. The QQ-plot shown in figure 2 indicates that the association signal is highly significant and that no undue inflation of p-value scores is observed for non-associated SNPs. The QQ-plot and Manhattan plots also indicate that the association signal extends to a large number of SNPs flanking the main association peak. Located near the very top of the association peak is the SNP (rs601338 p-value 2.98 x 10⁻²³) that underlies the functional change in the FUT2 gene. Figure 3 shows how the ¹H-NMR signal at 1.256 ppm varies as a function of the genotype at SNP rs601338.

Generation of P-value spectra for the identification of chemical compounds underlying a genotype-urine NMR-signal association.

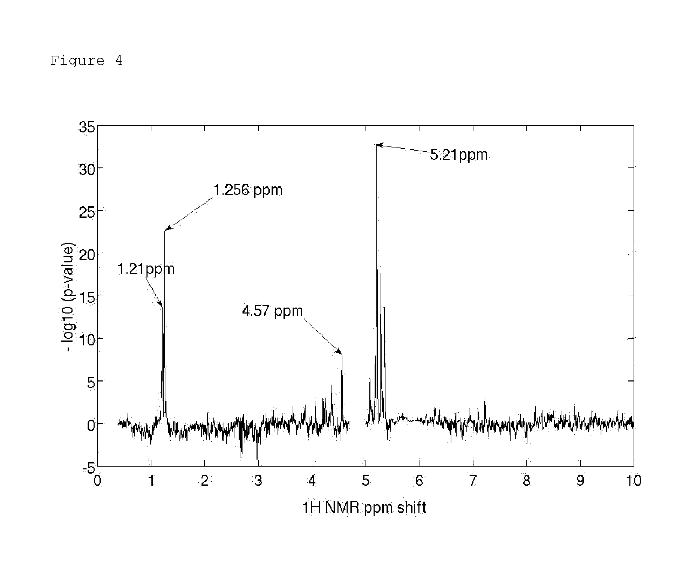

Analysis of the association patterns across different spectral bins indicated that certain genetic loci showed strong associations with not just one, but multiple spectral bins. Such multiple associations are not un-expected. A chemical compound underlying the association between a specific genetic locus and the signal intensity in a particular spectral bin could be expected to generate NMR-signals in other spectral bins as well. As a matter of fact, for a given genetic locus, the pattern of association signals (i.e. -log p-values) across the different spectral bins should resemble the NMR spectra of those chemical compounds that change concentrations as a function of the genotype at that locus. To track which of the compounds associated with a given genetic locus increase and which decrease as a function of a given genotype the -log p-values of association are multiplied by the slope (i.e. the beta) of the genotype-phenotype association signal. Figure4 shows the p-value spectra for the FUT2 locus (rs601338). The identified p-value spectrum recapitulates the main spectral lines (1.21ppm (d), 1.256 ppm (d), 4.57 ppm (d) 5.21 ppm (d)) found for pure L-fucose in the same sample matrix.

## Claims

1. Biomarker for gut health, wherein the biomarker is fucose, optionally bound fucose, such as fucose covalently linked to protein or sugar residues.

2. Biomarker in accordance with claim 1, wherein the fucose is L-fucose.

3. Biomarker in accordance with one of the preceding claims, wherein the fucose is free fucose.

4. Biomarker in accordance with one of the preceding claims, wherein the biomarker is to be detected in urine.

5. A method for determining the likelihood of having the FUT2 secretor genotype in a subject, comprising
- determining the level of fucose in a body fluid sample previously obtained from the subject to be tested, and
- comparing the subject's fucose level to a predetermined reference value,
wherein the predetermined reference value is based on an average body fluid fucose level in a control population, and wherein a lower body fluid fucose level in the sample compared to the predetermined reference value indicates an increased likelihood for a non-secretor FUT2 genotype and an increased body fluid fucose level indicates an increased likelihood for a secretor FUT2 genotype.

6. The method in accordance with claim 5, wherein the predetermined reference value is obtained from a control population with a non-secretor FUT2 genotype and a higher fucose level in the sample compared to the predetermined reference value indicates a secretor FUT2 genotype and an equal or lower fucose level indicates a non-secretor FUT2 genotype.

7. The method in accordance with one of claims 5 - 6, wherein the predetermined reference value is obtained from a control population with a secretor FUT2 genotype and a lower fucose level in the sample compared to the predetermined reference value indicates a non-secretor FUT2 genotype and an equal or higher fucose level indicates a secretor FUT2 genotype.

8. The method in accordance with one of claims 5-7, wherein a non-secretor FUT2 genotype corresponds to an increased risk for an impaired gut health, for example to an increased susceptibility to inflammatory bowel disease, Crohn's disease, or other chronic intestinal inflammatory processes.

9. The method in accordance with one of claims 5-8, wherein a non-secretor FUT2 genotype corresponds to an increased susceptibility to Type 1 Diabetes, and/or to an increased risk for metabolic deregulations.

10. The method in accordance with one of claims 5-9, wherein a non-secretor genotype corresponds to a decreased risk for infection by gastrointestinal viruses

11. The method in accordance with one of claims 5-10, wherein a non-secretor FUT2 genotype corresponds to an altered gut functional ecology and/or an increased risk towards gut dysbiosis.

12. The method in accordance with claim 10, wherein the altered gut functional ecology comprises reduced bifidobacterial diversity, reduced bifidobacterial richness, and/or reduced bifidobacterial abundance.

13. A method to stratify subjects according to their bifidobacterial population in the gut, comprising
- determining the level of fucose in a body fluid sample previously obtained from the subject to be tested, and
- comparing the subject's fucose level to a predetermined reference value,
wherein the predetermined reference value is based on an average body fluid fucose level in a control population, and wherein an equal or higher body fluid fucose level in the sample compared to the predetermined reference value indicates a rich bifidobacterial population in the gut, while a lower body fluid fucose level in the sample compared to the predetermined reference value indicates an impaired bifidobacterial population in the gut.

14. A method to test the effectiveness of medical or nutritional products in improving the gut microbiome, in particular in improving the bifidobacterial gut population in a subject, comprising
- determining the level of fucose in a body fluid sample previously obtained from the subject to be tested, and
- comparing the subject's fucose level to a predetermined reference value,
wherein the predetermined reference value is based on an average body fluid fucose level in a control population, and wherein a higher body fluid fucose level in the sample compared to the predetermined reference value indicates an improvement in the gut microbiome, in particular in the bifidobacterial gut population.

15. The method of claim 14, wherein the predetermined reference value is based on body fluid levels obtained from the subject before the administration of the medical or nutritional product has started.
